# EUROPÄISCHE PATENTSCHRIFT

(11) **EP 4 009 659 B1**
(45) Veröffentlichungstag und Bekanntmachung des Hinweises auf die Patenterteilung: **04.09.2024**
(21) Anmeldenummer: 20211095.3
(22) Anmeldetag: 01.12.2020
(51) Int. Cl.: H04R 1/10, A61F 11/14, H04M 1/60, H04M 1/05

(54) **GEHÖRSCHÜTZER MIT HEADSET-FUNKTION**
HEARING PROTECTION HEADSET
CASQUE MICRO ANTI-BRUIT

(43) Veröffentlichungstag der Anmeldung: 08.06.2022
(73) Patentinhaber: Andreas Stihl AG & Co. KG, 71336 Waiblingen (DE)
(72) Erfinder: Mang, Harald, 71364 Winnenden (DE); Conrad, Nils, 71404 Korb (DE); Langhammer, Maren, 71397 Leutenbach (DE); Danner, Julian, 71577 Großerlach (DE)
(74) Vertreter: Karzel, Philipp

(56) Entgegenhaltungen:
- HUSQVARNA: "HUSQVARNA ZUBEHÖR UND BEKLEIDUNG Katalog 2011 - Gehörschutz", 7 March 2011 (2011-03-07), XP055789824, Retrieved from the Internet <URL:https://issuu.com/husqvarna/docs/husqvarna_acc_2011_de> [retrieved on 20210325]
- HUSQVARNA: "Husqvarna-X-Com-R Hearing Protector Manual", 5 July 2019 (2019-07-05), XP055789837, Retrieved from the Internet <URL:https://www.manualslib.de/download/575441/Husqvarna-X-Com-R-Hp310-1.html> [retrieved on 20210325]
- HUSQVARNA: "Husqvarna Workshop manual Hearing Protector X-COM R", 16 April 2020 (2020-04-16), XP055789876, Retrieved from the Internet <URL:https://www.manualslib.com/manual/1789010/Husqvarna-X-Com-R.html> [retrieved on 20210325]

## Beschreibung

Die Erfindung betrifft ein Headset gemäß dem Oberbegriff des Anspruchs 1.

Aus der EP 3 451 689 A1 ist ein Headset bekannt, das zwei Hörmuscheln umfasst, wobei die Hörmuscheln über einen Tragbügel miteinander verbunden sind. Das Headset ist derart ausgebildet, dass der Bediener diese mittels des Tragbügels auf seinen Kopf klemmen kann und die Ohren des Bedieners durch die Hörmuscheln verdeckt sind. Zum einen dienen die Hörmuscheln als Gehörschutz, zum anderen sind in den Hörmuscheln Lautsprecher vorgesehen, um über eine Steuerschnittstelle Information an den Bediener zu übertragen. An den Hörmuscheln sind mehrere Tasten vorgesehen, die die Bedienung der Steuerschnittstelle ermöglichen.

Nachteilig an derartigen Headsets ist, dass die Tasten nur schwer auffindbar sind, wodurch die Bedienung der Steuerschnittstelle erschwert ist. Derartige Headsets sind insbesondere für Arbeiten im Freien vorgesehen, beispielsweise Waldarbeiten, Gartenarbeiten oder dgl., bei welchen der Bediener zumeist Arbeitshandschuhe trägt. Dieser Umstand erschwert zusätzlich das zielgerichtete Drücken der entsprechenden Tasten.

Die folgenden im Internet zugänglichen Dokumente offenbaren Headsets bei welchen mindestens ein Bedienelement in einer Vertiefung angeordnet ist:
"HUSQVARNA ZUBEHÖR UND BEKLEIDUNG Katalog 2011 - Gehörschutz", 7. März 2011, XP055789824, URL:https://issuu.com/husqvarna/docs/husqvarna_acc_2011_de [gefunden am 2021-03-25], sowie
"Husqvarna-X-Com-R Hearing Protector Manual", 5. Juli 2019, XP055789837, URL:https:// www.manualslib.de/download/575441/Husqvarna-X-Com-R-Hp310-1.html [gefunden am 2021-03-25].

Demnach liegt der Erfindung die Aufgabe zugrunde, ein Headset der gattungsgemäßen Art derart weiterzuentwickeln, dass eine einfache Bedienung des Headsets ermöglicht ist.

Diese Aufgabe wird durch ein Headset mit den Merkmalen des Anspruchs 1 gelöst.

Das Headset umfasst eine erste Hörmuschel und eine zweite Hörmuschel, wobei die erste Hörmuschel und die zweite Hörmuschel über ein Verbindungselement miteinander verbunden sind. Die Hörmuscheln weisen jeweils eine Innenseite und eine Außenseite auf, wobei die Innenseiten der Hörmuscheln einander zugewandt und die Außenseiten der Hörmuscheln einander abgewandt sind. Die Hörmuscheln erstrecken sich jeweils von ihrer Unterseite zu ihrer Oberseite in eine Hochrichtung und von ihrer ersten Längsseite zu ihrer zweiten Längsseite in eine zweite Querrichtung. Die erste Hörmuschel und/oder die zweite Hörmuschel weist an ihrer Außenseite eine Positioniertasche mit mindestens einer Taste auf, wobei sich die Positioniertasche über eine Breite in die zweite Querrichtung der Hörmuschel erstreckt, wobei die Breite der Positioniertasche mindestens 50% der in zweiter Querrichtung gemessenen Breite der Hörmuschel entspricht. Die Positioniertasche erstreckt sich in Hochrichtung der Hörmuschel von einem unteren Ende zu einem oberen Ende. Das obere Ende der Positioniertasche ist durch eine Anschlagkante zur Anlage der Fingerspitzen eines Bedieners ausgebildet.

Die Anschlagkante der Positioniertasche kann der Bediener auf einfache Weise ertasten. Das Headset ist dabei so ausgebildet, dass der Bediener seine Hand in die Positioniertasche rutschen lassen kann bis die Fingerspitzen des Bedieners an der Anschlagkante der Positioniertasche zur Anlage kommen. Liegen die Fingerspitzen des Bedieners in etwa an der Anschlagkante der Positioniertasche an, befindet sich die Hand in einer Grundstellung. Hierzu kann der Bediener seine Hand auf der Außenseite nahe der Unterseite der Hörmuschel positionieren und anschließend seine Hand in Hochrichtung zur Oberseite der Hörmuschel schieben, bis die Fingerspitzen die Anschlagkante der Positioniertasche kontaktieren. Dadurch kann die Hand des Bedieners auf einfache Weise immer wieder in die Grundstellung gebracht werden, von welcher aus die mindestens eine Taste in der Positioniertasche betätigt werden kann. Die Anschlagkante der Positioniertasche bildet somit ein einfach ertastbares Orientierungselement, wodurch die Bedienung des Headsets ohne Sichtkontakt mit dem Headset möglich ist.

Es ist vorzugsweise vorgesehen, dass die Anschlagkante eine in einer ersten Querrichtung von der Innenseite zur Außenseite der Hörmuschel gemessene Tiefe aufweist, wobei die Tiefe der Anschlagkante mindestens 5% der in erster Querrichtung gemessenen Tiefe der Hörmuschel entspricht. Diese Ausgestaltung der Anschlagkante ermöglicht ein noch einfacheres Ertasten der Anschlagkante. So ist es beispielsweise dem Bediener auch mit Handschuhen möglich, seine Hand durch Ertasten der Anschlagkante in die Grundstellung zu positionieren.

Die Positioniertasche geht an ihrem unteren Ende vorzugsweise absatzlos in eine die Außenseite der Hörmuschel bildende Grundfläche über. Durch den absatzlosen Übergang zwischen der Grundfläche der Hörmuschel und der Positioniertasche kann die Hand des Bedieners über die Grundfläche in die Positioniertasche geschoben werden.

Es ist vorteilhaft vorgesehen, dass die Positioniertasche in zweiter Querrichtung der Hörmuschel durch eine erste Seitenwand und eine zweite Seitenwand begrenzt ist, wobei die beiden Seitenwände eine Führung in Querrichtung der Hörmuschel zur seitlichen Führung der Finger des Bedieners bilden. Die erste Seitenwand und die zweite Seitenwand der Positioniertasche bilden Anschläge für die Finger des Bedieners in zweiter Querrichtung. Bereits beim Einschieben der Finger in die Positioniertasche werden diese durch die Seitenwände in Hochrichtung geführt. Befindet sich die Hand des Bedieners in Grundstellung, ist die Hand auf der Hörmuschel durch die Anschlagkante der Positioniertasche in Hochrichtung und durch die Seitenwände in zweiter Querrichtung ausgerichtet.

Es ist vorzugsweise vorgesehen, dass in der Positioniertasche der ersten Hörmuschel lediglich eine Verbindungstaste angeordnet ist, die sich über mindestens 50% der Breite der Positioniertasche erstreckt. Demnach ist die mindestens eine Taste der ersten Hörmuschel als Verbindungstaste ausgebildet. Durch die breite Ausgestaltung der Verbindungstaste kann diese durch den Bediener auf einfache Weise ertastet und betätigt werden. Die Verbindungstaste ist vorzugsweise zur Verbindung mit einer Rechnereinheit, insbesondere einem Smartphone, ausgebildet.

Es ist vorzugsweise vorgesehen, dass in der Positioniertasche der zweiten Hörmuschel mindestens zwei Tasten, insbesondere drei Tasten, angeordnet sind. Vorteilhaft sind die mindestens zwei Tasten respektive die mindestens drei Tasten durch einen Trennsteg voneinander getrennt. Dadurch kann der Bediener die Tasten auf einfache Weise voneinander unterscheiden. Vorzugsweise ist eine der mindestens zwei Tasten gegenüber der anderen Taste vertieft in Richtung zur Innenseite der Hörmuschel in der Positioniertasche angeordnet. Auch der Tiefenversatz der Tasten zueinander ermöglicht dem Bediener eine einfache Unterscheidung der Tasten. Vorteilhaft weist die mindestens eine Taste einen zumindest teilweise die Taste umlaufenden Kragen auf.

Es ist gemäß dem Anspruch 1 vorgesehen, dass an der ersten Hörmuschel und/oder an der zweiten Hörmuschel außerhalb der Positioniertasche eine Seitentaste angeordnet ist, wobei die Seitentaste und die Positioniertasche derart zueinander angeordnet sind, dass in einer Grundstellung der Hand des Bedieners die mindestens eine Taste in der Positioniertasche und die Seitentaste betätigbar sind, ohne die Hand neu positionieren zu müssen. Vorzugsweise sind in der Grundstellung der Hand des Bedieners drei Tasten in der Positioniertasche und die Seitentaste betätigbar, ohne die Hand neu positionieren zu müssen. Dadurch ist eine besonders ergonomische Bedienung des Headsets möglich.

Zudem ist gemäß dem Anspruch 1 die Seitentaste zwischen der Unterseite der Hörmuschel und der zweiten Längsseite der Hörmuschel angeordnet. Dadurch lässt sich die Seitentaste mit dem Daumen auf einfache Weise betätigen.

Es ist vorteilhaft vorgesehen, dass in Blickrichtung entlang der ersten Querrichtung auf die Außenseite die Hörmuschel einen ersten Flächenschwerpunkt und die Seitentaste einen zweiten Flächenschwerpunkt aufweist, wobei der in Hochrichtung gemessene Abstand zwischen dem ersten Flächenschwerpunkt und dem zweiten Flächenschwerpunkt mindestens 20%, insbesondere mindestens 30%, vorzugsweise in etwa 35% der Höhe der Hörmuschel entspricht. Der erste Flächenschwerpunkt der Hörmuschel liegt vorzugsweise auf der Positioniertasche, insbesondere auf der mindestens einen Taste. Durch den Abstand zwischen den zwei Flächenschwerpunkten wird eine ergonomische Anordnung der Seitentaste zu der mindestens einen Taste in der Positioniertasche gewährleistet. Dies ermöglicht dem Bediener, in der Grundstellung der Hand die mindestens eine Taste mit dem Zeigefinger, Mittelfinger und/oder dem Ringfinger betätigen zu können, und zudem in der Grundstellung auf komfortable Weise mit dem Daumen auch die Seitentaste betätigen zu können.

Vorteilhaft weist die Seitentaste der ersten Hörmuschel drei noppenartige Erhöhungen auf. Dadurch lässt sich die Seitentaste der ersten Hörmuschel von der Seitentaste der zweiten Hörmuschel unterscheiden.

Vorzugsweise ist die Seitentaste der zweiten Hörmuschel als eine kreisförmige Funktions-Taste, insbesondere als eine push-to-talk-Taste oder eine Stummschaltungs-Taste, ausgebildet. Mit Betätigung der Seitentaste der ersten Hörmuschel oder der Seitentaste der zweiten Hörmuschel kann eine kommunikationsbezogene Funktion aktiviert oder deaktiviert werden, insbesondere eine push-to-talk Funktion für ein mit dem Headset verbindbares Funkgerät oder eine Funktion für eine Gruppenkommunikation oder eine Mikrofon-Stummschaltung.

Weitere Merkmale der Erfindung ergeben sich aus der Beschreibung und der Zeichnung, in der ein nachfolgend im Einzelnen beschriebenes Ausführungsbeispiel der Erfindung dargestellt ist. Es zeigen:
- Fig. 1: in perspektivischer Darstellung auf die erste Hörmuschel ein erfindungsgemäßes Headset,
- Fig. 2: in perspektivischer Darstellung auf die zweite Hörmuschel das Headset nach Fig. 1,
- Fig. 3: in Vorderansicht das Headset nach Fig. 1,
- Fig. 4: in Seitenansicht auf die erste Hörmuschel das Headset nach Fig. 1,
- Fig. 5: in Seitenansicht auf die zweite Hörmuschel das Headset nach Fig. 1,
- Fig. 6: in Seitenansicht eine ausschnittsweise, schematische Darstellung der ersten Hörmuschel ohne Taste und
- Fig. 7: in ausschnittsweiser Seitenansicht auf die erste Hörmuschel das Headset nach Fig. 1.

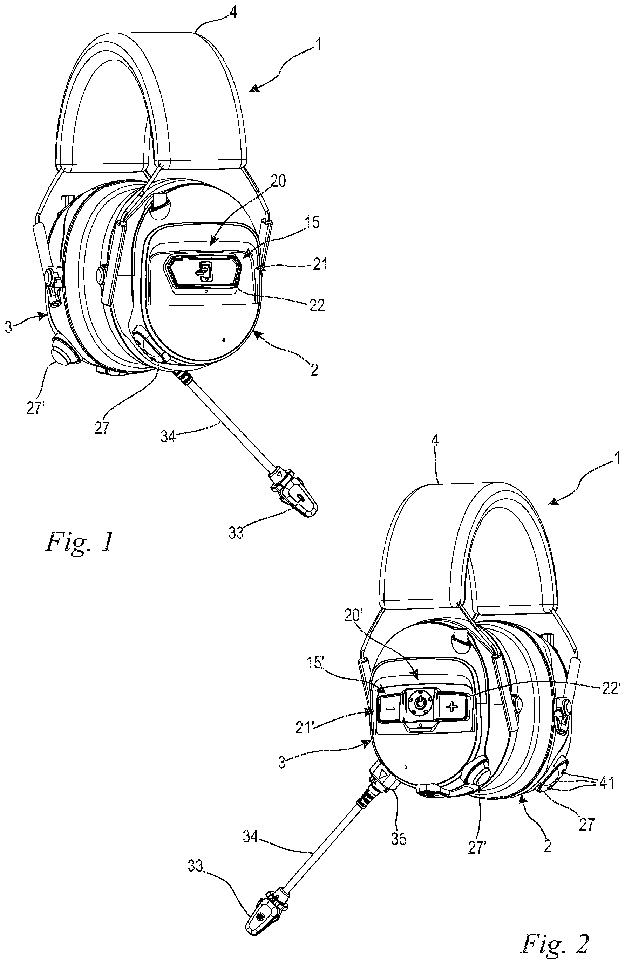

In Fig. 1 ist ein erfindungsgemäßes Headset 1 gezeigt. Das Headset 1 dient unter anderem als Gehörschutz bei Arbeiten mit entsprechend hohen Geräuschemissionen. Hierzu zählen beispielsweise Arbeiten mit Motorkettensägen, Freischneidern, Trimmern, Blasgeräten, Trennschleifern, Gesteinsschneidern und dgl. Das Headset 1 umfasst eine erste Hörmuschel 2, eine zweite Hörmuschel 3 und ein Verbindungselement 4. Die erste Hörmuschel 2 und die zweite Hörmuschel 3 sind über das Verbindungselement 4 miteinander verbunden. In dem vorliegenden Ausführungsbeispiel ist das Verbindungselement 4 als ein elastischer Bügel ausgebildet. Der Bügel ist zumindest teilweise mit einem Polster zur Erhöhung des Tragekomforts des Headsets 1 umkleidet. In einer alternativen, nicht dargestellten Ausführung des Headsets 1 kann das Verbindungselement 4 auch als ein Schutzhelm oder dgl. ausgebildet sein. In einer solchen Ausführung sind die erste Hörmuschel 2 und die zweite Hörmuschel 3 über Befestigungselemente an dem Schutzhelm gehalten.

Die erste Hörmuschel 2 sowie die zweite Hörmuschel 3 umfassen jeweils einen nicht dargestellten Lautsprecher, wobei der Lautsprecher jeweils in den Hörmuscheln 2, 3 angeordnet ist. Zudem umfasst das Headset 1 ein Mikrofon 33, das über einen Haltebügel 34 an einer der beiden Hörmuscheln 2, 3 befestigt ist. Der Haltebügel 34 ist vorzugsweise an der zweiten Hörmuschel 3 befestigt. Es kann jedoch auch zweckmäßig sein, den Haltebügel 34 mit Mikrofon 33 an der ersten Hörmuschel 2 zu befestigen. Vorzugsweise sind an beiden Hörmuscheln 2, 3 Aufnahmen 35 für den Haltebügel 34 vorgesehen, so dass der Bediener den Haltebügel 34 entweder an der ersten Hörmuschel 2 oder an der zweiten Hörmuschel 3 befestigen kann. Der Haltebügel 34 ist derart gestaltet, dass dieser das Mikrofon 33 näher an den Mund des Bedieners hält, wenn der Bediener das Headset 1 wie vorgesehen trägt.

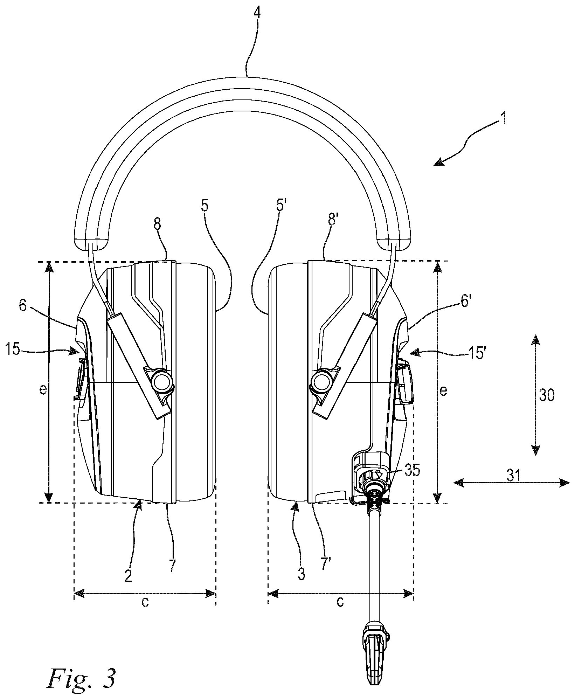

Wie in Fig. 3 gezeigt, weisen die erste Hörmuschel 2 und die zweite Hörmuschel 3 jeweils eine Außenseite 6, 6' und jeweils eine Innenseite 5, 5' auf. Die Innenseiten 5, 5' der Hörmuscheln 2, 3 sind einander zugewandt, die Außenseiten 6, 6' der Hörmuscheln 2, 3 sind einander abgewandt. Die Hörmuscheln 2, 3 erstrecken sich jeweils von ihrer Innenseite 5, 5' zur Außenseite 6, 6' in eine erste Querrichtung 31.

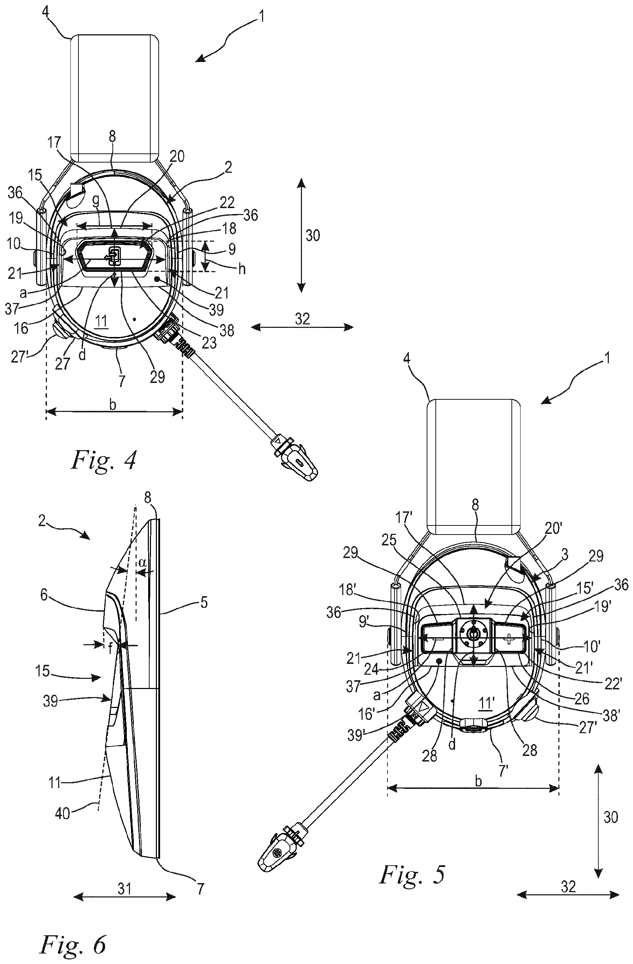

Wie in den Figuren 4 und 5 gezeigt, ist die Hörmuschel 2, 3 im Ausführungsbeispiel in Blickrichtung der Figuren 4 und 5, also in Blickrichtung der ersten Querrichtung 31, in etwa ovalförmig ausgebildet. Auch andere Formen der Hörmuschel 2, 3 können zweckmäßig sein. Der Hörmuschel 2, 3 sind wie folgt einzelne Seiten zuzuweisen:
Wie in Fig. 3 gezeigt, erstrecken sich die Hörmuscheln 2, 3 von ihrer Unterseite 7, 7' zur Oberseite 8, 8' in eine Hochrichtung 30. Die Oberseite 8, 8' ist die dem Haltebügel 34 abgewandte Seite der Hörmuschel 2, 3, die Unterseite 7, 7' ist die dem Haltebügel 34 zugewandte Seite der Hörmuschel 2, 3. Wie in den Figuren 4 und 5 gezeigt, erstecken sich die Hörmuscheln 2, 3 von einer ersten Längsseite 9, 9' zur zweiten Längsseite 10, 10' in eine zweite Querrichtung 32. Die erste Längsseite 9, 9' der Hörmuschel 2, 3 ist die dem Mikrofon 33 zugewandte Seite, die zweite Längsseite 10, 10' der Hörmuschel 2, 3 ist die dem Mikrofon 33 abgewandte Seite. Im Ausführungsbeispiel sind die erste Querrichtung 31, die zweite Querrichtung 32 und die Hochrichtung 30 des Headsets 1 jeweils senkrecht zueinander ausgerichtet. Damit entsprechen die erste Querrichtung 31, die zweite Querrichtung 32 und die Hochrichtung 30 zumindest im Hinblick auf deren Ausrichtung einem geradlinigen, orthogonalen Koordinatensystem (kartesisches Koordinatensystem).

Wie in den Figuren 1 und 2 gezeigt, umfassen im erfindungsgemäßen Ausführungsbeispiel des Headsets 1 beide Hörmuscheln 2, 3 jeweils eine Positioniertasche 15, 15' zur einfachen Positionierung der Hand des Bedieners. Es kann auch zweckmäßig sein, dass lediglich die erste Hörmuschel 2 oder die zweite Hörmuschel 3 eine Positioniertasche 15, 15' umfasst. Die Positioniertasche 15, 15' ist auf der Außenseite 6, 6' der Hörmuschel 2, 3 angeordnet. In der Positioniertasche 15, 15' ist mindestens eine Taste 22, 22' angeordnet.

Wie in den Figuren 4 und 5 gezeigt, umfasst die Positioniertasche 15, 15' eine Anschlagkante 20, 20'. Die Anschlagkante 20, 20' erstreckt sich in die zweite Querrichtung 32. Die Anschlagkante 20, 20' ist derart ausgebildet, dass die Fingerspitzen beim Einführen in die Positioniertasche 15, 15' in Hochrichtung 30 die Anschlagkante 20, 20' kontaktieren und an dieser anschlagen. Ferner umfasst die Positioniertasche 15, 15' eine erste Seitenwand 18, 18' und eine zweite Seitenwand 19, 19'. Die Seitenwände 18, 18', 19, 19' erstrecken sich in etwa in Hochrichtung 30. Die erste Seitenwand 18, 18' und die zweite Seitenwand 19, 19' weisen jeweils ein der Oberseite 8, 8' der Hörmuschel 2, 3 zugewandtes erstes Ende 36 auf. Die erste Seitenwand 18, 18' und die zweite Seitenwand 19, 19' sind über die Anschlagkante 20, 20' miteinander verbunden. Die Anschlagkante 20, 20' schließt an den ersten Enden 36 der beiden Seitenwände 18, 18', 19, 19' an. Die Seitenwände 18, 18', 19, 19' bilden eine Führung 21, 21' für die Finger des Bedieners in Hochrichtung 30. Durch die Führung 21, 21' sind die Finger in zweiter Querrichtung 32 positioniert. Zudem wird ein Abrutschen der Finger in zweiter Querrichtung 32 vermieden.

Wie in den Figuren 4 und 5 gezeigt, besitzt die Positioniertasche 15, 15' eine Breite a, die dem in zweiter Querrichtung 32 gemessenen Abstand der beiden Seitenwände 18, 18', 19, 19' entspricht. Die Hörmuschel 2, 3 besitzt eine Breite b, die dem in zweiter Querrichtung 32 gemessenen maximalen Abstand zwischen der ersten Längsseite 9, 9' und der zweiten Längsseite 10, 10' der Hörmuschel 2, 3 entspricht. Die Breite a der Positioniertasche 15 beträgt mindestens 50%, insbesondere mindestens 60%, vorzugsweise in etwa 75% der Breite b der Hörmuschel 2, 3.

Wie in den Figuren 4 und 5 gezeigt, umfasst die Positioniertasche 15, 15' eine Höhe d, die dem in Hochrichtung 30 gemessenen Abstand zwischen einem unteren Ende 16, 16' und einem oberen Ende 17, 17' der Positioniertasche 15 entspricht. Das obere Ende 17, 17' der Positioniertasche 15, 15' ist durch die Anschlagkante 20, 20' der Positioniertasche 15, 15' gebildet. Das untere Ende 16, 16' der Positioniertasche 15, 15' ist der Abschnitt der Hörmuschel 2, 3, in dem die Positioniertasche 15, 15' in eine die Außenseite 6, 6' bildende Grundfläche 11, 11' der Hörmuschel 2, 3 übergeht. Im vorliegenden Ausführungsbeispiel ist dieser Abschnitt durch eine absatzlose Kante 38, 38' zwischen einer Bodenfläche 39, 39' der Positioniertasche 15, 15' und der Grundfläche 11, 11' der Hörmuschel 2, 3 gebildet. Es kann auch zweckmäßig sein, anstelle der Kante 38, 38' einen fließenden Übergang zwischen der Bodenfläche 39, 39' der Positioniertasche 15, 15' und der Grundfläche 11, 11' der Hörmuschel 2, 3 vorzusehen. Zudem ist im Ausführungsbeispiel das untere Ende 16, 16' der Positioniertasche 15, 15' auch durch die der Unterseite 7, 7' der Hörmuschel 2, 3 zugewandten zweiten Enden 37 der Seitenwände 18, 18', 19, 19' der Positioniertasche 15, 15' gebildet. Die Hörmuschel 2, 3 besitzt eine Höhe e, die dem in Hochrichtung 30 gemessenen maximalen Abstand zwischen der Unterseite 7, 7' und der Oberseite 8, 8' der Hörmuschel 2, 3 entspricht. Die Höhe d der Positioniertasche 15, 15' beträgt mindestens 15%, insbesondere mindestens 25%, vorzugsweise in etwa 30% der Höhe e der Hörmuschel 2, 3.

Im Ausführungsbeispiel ist die Bodenfläche 39, 39' der Positioniertasche 15, 15' leicht konvex gekrümmt ausgebildet. Wie in Fig. 6 schematisch dargestellt, ist eine an die Bodenfläche 39, 39' der Positioniertasche 15, 15' angenäherte Näherungsebene 40 gezeigt. In der Seitenansicht der ersten Hörmuschel 2 nach Fig. 6 wurde auf die Darstellung einer Taste 22 verzichtet, um die Bodenfläche 39 besser darstellen zu können. Die Näherungsebene 40 schließt mit der Innenseite 5, 5' der Hörmuschel 2, 3 einen Winkel α ein, der zur Unterseite 7, 7' der Hörmuschel 2, 3 hin geöffnet ist. Im Ausführungsbeispiel beträgt der Winkel α mindestens 5°, insbesondere in etwa 7°. Es kann in einer alternativen Ausführung auch zweckmäßig sein, die Bodenfläche 39, 39` bzw. deren Näherungsebene 40 und die Innenseite 5, 5' der Hörmuschel 2, 3 in etwa parallel zueinander auszurichten.

Wie in Fig. 6 gezeigt, weist die Anschlagkante 20, 20' eine Tiefe f auf, die dem in erster Querrichtung 31 gemessenen maximalen Abstand zwischen der Grundfläche 11 der Hörmuschel 2, 3 und der Bodenfläche 39, 39' der Positioniertasche 15, 15' entspricht. Die Hörmuschel 2, 3 weist eine Tiefe c (Fig. 3) auf, die dem in erster Querrichtung 31 gemessenen maximalen Abstand zwischen der Innenseite 5, 5' und der Außenseite 6, 6' der Hörmuschel 2, 3 entspricht. Im Ausführungsbeispiel beträgt die Tiefe f der Anschlagkante 20, 20' mindestens 5% der Tiefe c der Hörmuschel 2, 3.

Das Headset 1 umfasst ferner eine nicht dargestellte Steuerschnittstelle, um verschiedene Gerätschaften über das Headset 1 ansteuern zu können oder Funktionen des Headsets 1 ausführen zu können. Über die Steuerschnittstelle ist das Headset 1 mit einer Rechnereinheit, insbesondere mit einem Smartphone, einem Tablet, einem Notebook oder dgl. verbindbar. Ferner umfasst das Ausführungsbeispiel des Headsets 1 ein Radio, einen Musikspieler und/oder dgl. In der bevorzugten Ausführungsform ist das Headset 1 zum Verbinden mit einem Funkgerät ausgebildet.

Wie in Fig. 4 gezeigt, ist in der Positioniertasche 15, 15' der ersten Hörmuschel 2, 3 lediglich eine Taste 22 angeordnet. Die Taste 22 ist als eine Verbindungstaste 23 ausgebildet. Die Verbindungstaste 23 ermöglicht es dem Bediener, das Headset 1 mit der Rechnereinheit, insbesondere mit einem Smartphone, Tablet, Notebook oder dgl. zu verbinden. Bevorzugt ist über die Verbindungstaste 23 auch das Radio aktivierbar. Ist das Headset 1 mit der Rechnereinheit verbunden, können vorzugsweise über die Verbindungstaste 23 Anrufe entgegengenommen und beendet werden und Sprachassistenten zur Bedienung weiterer Applikationen aktiviert werden. Es kann vorteilhaft sein, die Verbindungstaste 23 mit weiteren Funktionen zu belegen.

Wie in Fig. 4 gezeigt, umfasst die Verbindungstaste 23 eine in zweiter Querrichtung 32 gemessene Breite g, die mindestens 50%, insbesondere mindestens 60%, vorzugsweise in etwa 75% der Breite a der Positioniertasche 15, 15' entspricht. Ferner entspricht die Breite g der Verbindungstaste 23 mindestens 25%, vorzugsweise mindestens 35%, insbesondere mindestens 45% der Breite b der Hörmuschel 2, 3. Die Verbindungstaste 23 umfasst eine in Hochrichtung 30 gemessene Höhe h, die mindestens 30%, insbesondere mindestens 40%, vorzugsweise in etwa 50% der Höhe d der Positioniertasche 15, 15' entspricht. Im Ausführungsbeispiel umfasst die Verbindungstaste 23 einen die Verbindungstaste 23 umlaufenden, hochgestellten Kragen 29. Der Kragen 29 ermöglicht ein einfaches Ertasten der Verbindungstaste 23.

Wie in Fig. 4 gezeigt, umfasst die erste Hörmuschel 2 eine Seitentaste 27. Die Seitentaste 27 der ersten Hörmuschel 2 ist zwischen der Unterseite 7 der ersten Hörmuschel 2 und der zweiten Längsseite 10 der Hörmuschel 2 angeordnet. Auf der Seitentaste 27 der ersten Hörmuschel 2 sind drei noppenartige Erhöhungen 41 angebracht. Im Ausführungsbeispiel sind die Erhöhungen in einer Reihe angeordnet. Die noppenartigen Erhöhungen 41 ermöglichen es dem Bediener, die Seitentaste 27 auf einfache Weise zu erkennen. Im bevorzugten Ausführungsbeispiel des Headsets 1 kann über die Seitentaste 27 der ersten Hörmuschel 2 das Mikrofon 33 aktiviert und deaktiviert werden. Vorzugsweise kann auch eine Gruppenkommunikation mit anderen Bedienern, die ein solches Headset 1 tragen, durch Schalten des Mikrofons 33 aktiviert und deaktiviert werden. Die Seitentaste 27 ist mit der Positioniertasche 15 sowie der entsprechenden Verbindungstaste 23 derart angeordnet, dass nach Auflegen der Hand des Bedieners in die Grundstellung der Bediener die Verbindungstaste 23 mit seinem Zeigefinger, Mittelfinger und/oder Ringfinger und die Seitentaste 27 mit dem Daumen betätigen kann. Die Hand muss dabei nicht verschoben werden. Dadurch wird eine ergonomische Bedienung der ersten Hörmuschel 2 gewährleistet. Die Hand des Bedieners befindet sich dann in der Grundstellung, wenn die Fingerspitzen des Bedieners in etwa an der Anschlagkante 20, 20' der Positioniertasche 15, 15' anliegen.

Wie in Fig. 5 gezeigt, sind in der Positioniertasche 15' der zweiten Hörmuschel 3 drei Tasten 24, 25, 26 angeordnet. Die drei Tasten 24, 25, 26 sind eine erste Taste 24, eine zweite Taste 25 und eine dritte Taste 26. Die drei Tasten 24, 25, 26 sind in der zweiten Querrichtung 32 nebeneinander angeordnet. Dadurch kann der Bediener in der Grundstellung seiner Hand auf jede der drei Tasten 24, 25, 26 einen Finger ablegen, wodurch die Bedienung der Tasten 24, 25, 26 vereinfacht ist. Die zweite Taste 25 ist zwischen der ersten Taste 24 und der dritten Taste 26 angeordnet. In einer alternativen Ausführung kann es auch zweckmäßig sein, eine andere Anzahl Tasten in der Positioniertasche 15' vorzusehen. Die zweite Taste 25 ist gegenüber der ersten Taste 24 und der dritten Taste 26 in erster Querrichtung 31 versetzt, insbesondere erhöht angeordnet. Die zweite Taste 25 ist jeweils durch einen Trennsteg 28 von der ersten Taste 24 und der dritten Taste 26 getrennt. Der Trennsteg 28 erlaubt dem Bediener, die drei Tasten 24, 25, 26 in ihrer räumlichen Anordnung haptisch auf einfache Weise voneinander unterscheiden zu können. Zudem sind die erste Taste 24 und die dritte Taste 26 an ihrem Umfang durch einen aufgestellten Kragen 29 zumindest teilweise umrandet. Der Kragen 29 der ersten Taste 24 und der dritten Taste 26 schließt jeweils an dem Trennsteg 28 an. Der Kragen 29 der ersten Taste 24 und der dritten Taste 26 ermöglicht dem Bediener eine einfache Erkennung der Tasten.

Im Ausführungsbeispiel ist über die erste Taste 24 und die dritte Taste 26 vorzugsweise die Lautstärke der Lautsprecher in den beiden Hörmuscheln 2, 3 einzustellen. Zudem kann mittels der dritten Taste 26 das Radio aktiviert und deaktiviert werden. Mittels der zweiten Taste 25 ist das Headset 1 einschaltbar. Vorzugsweise kann über die zweite Taste 25 das Headset 1 auch mit der Rechnereinheit, also einem Smartphone und dgl., gekoppelt werden. Ferner dient die zweite Taste 25 im Ausführungsbeispiel vorzugsweise auch zur Navigation bei der Musikwiedergabe sowie zur Senderwahl bei der Nutzung des Radios.

Wie in Fig. 5 gezeigt, umfasst die zweite Hörmuschel 3 eine Seitentaste 27`. Die Seitentaste 27' der zweiten Hörmuschel 3 ist zwischen der Unterseite 7' der zweiten Hörmuschel 3 und der ersten Längsseite 9' der zweiten Hörmuschel 3 angeordnet. Die Seitentaste 27' besitzt im Ausführungsbeispiel eine kreisrunde Grundform (Fig. 1). Dadurch, dass die Seitentaste 27 der ersten Hörmuschel 2 und die Seitentaste 27' der zweiten Hörmuschel 3 verschiedenartig ausgebildet sind, sind beide Seitentasten 27, 27' für den Bediener einfach zu unterscheiden. Im bevorzugten Ausführungsbeispiel des Headsets 1 ist die Seitentaste 27' als eine kommunikationsbezogene Funktions-Taste, insbesondere eine push-to-talk-Taste oder eine Stummschaltungs-Taste, ausgebildet.

Wie in Fig. 5 gezeigt, sind die Seitentaste 27' der zweiten Hörmuschel 3 und die Tasten 24, 25, 26 in der Positioniertasche 15' derart angeordnet, dass aus der Grundstellung der Hand des Bedieners die drei Tasten 24, 25, 26 sowie die Seitentaste 27 betätigbar sind. Vorzugsweise ist in der Grundstellung der Hand die erste Taste 24 über den Ringfinger, die zweite Taste 25 über den Mittelfinger, die dritte Taste 26 über den Zeigefinger und die Seitentaste 27' über den Daumen betätigbar. Somit können aus der Grundstellung der Hand alle Tasten betätigt werden, ohne dabei die Hand neu positionieren zu müssen.

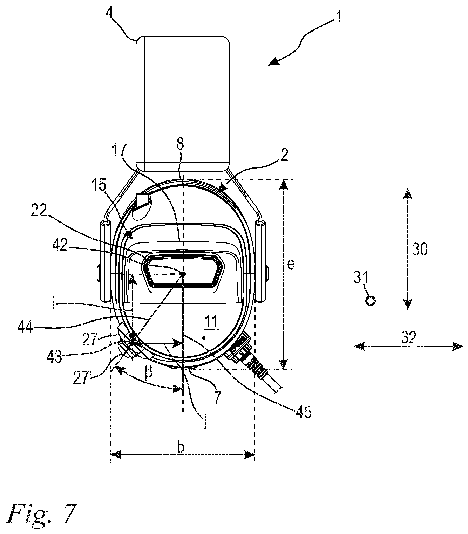

In Fig. 7 ist das erfindungsgemäße Headset 1 in einer ausschnittsweisen Seitendarstellung auf die erste Hörmuschel 2 gezeigt. Die erste Hörmuschel 2 weist in Blickrichtung entlang der ersten Querrichtung 31 gerichtet auf die Außenseite 6 einen ersten Flächenschwerpunkt 42 auf. Der erste Flächenschwerpunkt 42 ist im Ausführungsbeispiel, insbesondere in etwa mittig, in der Positioniertasche 15 angeordnet. Der erste Flächenschwerpunkt 42 ist im Ausführungsbeispiel, insbesondere in etwa mittig, in der mindestens einen Taste 22 angeordnet. Die Seitentaste 27 der Hörmuschel 2 weist in Blickrichtung entlang der ersten Querrichtung 31 gerichtet auf die Außenseite 6 einen zweiten Flächenschwerpunkt 43 auf. Der erste Flächenschwerpunkt 42 der ersten Hörmuschel 2 und der zweite Flächenschwerpunkt 43 der Seitentaste 27 weisen einen in Hochrichtung 30 gemessenen Abstand i auf, wobei der Abstand i mindestens 20%, insbesondere mindestens 30%, vorzugsweise in etwa 35% der Höhe e der Hörmuschel 2 entspricht. Der erste Flächenschwerpunkt 42 der ersten Hörmuschel 2 und der zweite Flächenschwerpunkt 43 der Seitentaste 27 weisen einen in zweiter Querrichtung 32 gemessenen Abstand j auf, wobei der Abstand j mindestens 20%, insbesondere mindestens 30%, vorzugsweise in etwa 35% der Breite b der Hörmuschel 2 entspricht. Wie in Fig. 7 gezeigt, verläuft durch den ersten Flächenschwerpunkt 42 der Hörmuschel 2 eine in Hochrichtung 30 ausgerichtete Längsgerade 45. Ferner ist eine Verbindungsgerade 44 gezeigt, die durch den ersten Flächenpunkt 42 und durch den zweiten Flächenpunkt 43 verläuft und zugleich die Längsgerade 45 schneidet. Wie in Fig. 7 gezeigt, spannen die Längsgerade 45 und die Verbindungsgerade 44 einen Winkel β auf, wobei der Winkel β zur Unterseite 7 der ersten Hörmuschel 2 hin geöffnet ist. Der Winkel β liegt im Ausführungsbeispiel in einem Bereich von 15° bis 60°, vorzugsweise von 25° bis 45°. Der Winkel β beträgt vorzugsweise in etwa 35°.

Die erste Hörmuschel 2 und die zweite Hörmuschel 3 sind bezüglich ihrer Grundform im Wesentlichen gegenüber einer durch die Hochrichtung 30 und die zweite Querrichtung 32 aufgespannten Ebene spiegelsymmetrisch ausgebildet. Daher gelten die oben beschriebenen Verhältnisse der Anordnung der Seitentaste 27 zum Flächenschwerpunkt 42 der ersten Hörmuschel 2 auch für die zweite Hörmuschel 3.

## Patentansprüche

1. Headset,
umfassend eine erste Hörmuschel (2) und eine zweite Hörmuschel (3), wobei die erste Hörmuschel (2) und die zweite Hörmuschel (3) über ein Verbindungselement (4) miteinander verbunden sind,
wobei die Hörmuscheln (2, 3) jeweils eine Innenseite (5, 5') und eine Außenseite (6, 6') aufweisen, wobei die Innenseiten (5, 5') der Hörmuscheln (2, 3) einander zugewandt und die Außenseiten (6, 6') der Hörmuscheln (2, 3) einander abgewandt sind,
wobei sich die Hörmuscheln (2, 3) jeweils von ihrer Unterseite (7, 7') zu ihrer Oberseite (8, 8') in eine Hochrichtung (30) und von ihrer ersten Längsseite (9, 9') zu ihrer zweiten Längsseite (10, 10') in eine zweite Querrichtung (32) erstrecken, wobei die erste Hörmuschel (2) und/oder die zweite Hörmuschel (3) an ihrer Außenseite (6, 6') eine Positioniertasche (15, 15') mit mindestens einer Taste (22, 22') aufweist, wobei sich die Positioniertasche (15, 15') über eine Breite (a) in zweiter Querrichtung (32) der Hörmuschel (2, 3) erstreckt, wobei die Breite (a) der Positioniertasche (15, 15') mindestens 50% der in zweiter Querrichtung gemessenen Breite (b) der Hörmuschel (2, 3) entspricht,
wobei sich die Positioniertasche (15, 15') in Hochrichtung (30) der Hörmuschel (2, 3) von einem unteren Ende (16, 16') zu einem oberen Ende (17, 17') erstreckt, das obere Ende (17, 17') der Positioniertasche (15, 15') durch eine Anschlagkante (20, 20') zur Anlage der Fingerspitzen eines Bedieners ausgebildet ist,
**dadurch gekennzeichnet, dass** an der ersten Hörmuschel (2) und/oder an der zweiten Hörmuschel (3) außerhalb der Positioniertasche (15, 15') eine Seitentaste (27, 27') angeordnet ist, wobei die Seitentaste (27, 27') und die Positioniertasche (15, 15') derart zueinander angeordnet sind, dass in einer Grundstellung der Hand des Bedieners die mindestens eine Taste (22, 22') in der Positioniertasche (15, 15') und die Seitentaste (27, 27') betätigbar sind, ohne die Hand neu positionieren zu müssen, und dass
die Seitentaste (27, 27') zwischen der Unterseite (7, 7') der Hörmuschel (2, 3) und der zweiten Längsseite (10, 10') der Hörmuschel (2, 3) angeordnet ist.

2. Headset nach Anspruch 1,
**dadurch gekennzeichnet, dass** die Anschlagkante (20, 20') eine in einer ersten Querrichtung von der Innenseite (5, 5') zur Außenseite (6, 6') der Hörmuschel (2, 3) gemessene Tiefe (f) aufweist, wobei die Tiefe (f) der Anschlagkante (20, 20') mindestens 5% der in erster Querrichtung (31) gemessenen Tiefe (c) der Hörmuschel (2, 3) entspricht.

3. Headset nach Anspruch 1 oder 2,
**dadurch gekennzeichnet, dass** die Positioniertasche (15, 15') an ihrem unteren Ende (16, 16') absatzlos in eine die Außenseite (6, 6') der Hörmuschel (2, 3) bildende Grundfläche (11, 11') übergeht.

4. Headset nach einem der Ansprüche 1 bis 3,
**dadurch gekennzeichnet, dass** die Positioniertasche (15, 15') in zweiter Querrichtung (32) der Hörmuschel (2, 3) durch eine erste Seitenwand (18, 18') und eine zweite Seitenwand (19, 19') begrenzt ist, wobei die beiden Seitenwände (18, 18', 19, 19') eine Führung (21, 21') in zweiter Querrichtung (32) der Hörmuschel (2, 3) zur seitlichen Führung der Finger des Bedieners bilden.

5. Headset nach einem der Ansprüche 1 bis 4,
**dadurch gekennzeichnet, dass** in der Positioniertasche (15, 15') der ersten Hörmuschel (2) lediglich eine Verbindungstaste (23) angeordnet ist, die sich über mindestens 50% der Breite (a) der Positioniertasche (15, 15') erstreckt.

6. Headset nach Anspruch 5,
**dadurch gekennzeichnet, dass** die Verbindungstaste (23) zur Verbindung mit einer Rechnereinheit, insbesondere einem Smartphone, ausgebildet ist.

7. Headset nach einem der Ansprüche 1 bis 6,
**dadurch gekennzeichnet, dass** in der Positioniertasche (15, 15') der zweiten Hörmuschel (3) mindestens zwei Tasten (24, 25, 26), insbesondere drei Tasten (24, 25, 26), angeordnet sind.

8. Headset nach Anspruch 7,
**dadurch gekennzeichnet, dass** die mindestens zwei Tasten (24, 25, 26) durch einen Trennsteg (28) voneinander getrennt sind.

9. Headset nach Anspruch 7 oder 8,
**dadurch gekennzeichnet, dass** eine der mindestens zwei Tasten (24, 25, 26) gegenüber der anderen Taste (24, 25, 26) vertieft in Richtung zur Innenseite (5, 5') der Hörmuschel (3) in der Positioniertasche (15, 15') angeordnet ist.

10. Headset nach Anspruch 9,
**dadurch gekennzeichnet, dass** in der Grundstellung der Hand des Bedieners drei in der Positioniertasche (15, 15') angeordnete Tasten (24, 25, 26) und die Seitentaste (27') betätigbar sind, ohne die Hand neu positionieren zu müssen.

11. Headset nach einem der Ansprüche 1 bis 10,
**dadurch gekennzeichnet, dass** in Blickrichtung entlang der ersten Querrichtung (31) auf die Außenseite (6, 6') die Hörmuschel (2, 3) einen ersten Flächenschwerpunkt (42) und die Seitentaste (27, 27') einen zweiten Flächenschwerpunkt (43) aufweist, wobei der in Hochrichtung (30) gemessene Abstand (i) zwischen dem ersten Flächenschwerpunkt (42) und dem zweiten Flächenschwerpunkt (43) mindestens 20%, insbesondere mindestens 30%, vorzugsweise in etwa 35% der Höhe (e) der Hörmuschel (2, 3) entspricht.

12. Headset nach einem der Ansprüche 1 bis 11,
**dadurch gekennzeichnet, dass** die Seitentaste (27) der ersten Hörmuschel (2) drei noppenartige Erhöhungen (41) aufweist.

13. Headset nach einem der Ansprüche 1 bis 12,
**dadurch gekennzeichnet, dass** die Seitentaste (27') der zweiten Hörmuschel (3) als eine kreisförmige Funktions-Taste, insbesondere eine push-to-talk-Taste oder eine Stummschaltungs-Taste, ausgebildet ist.

## Claims

1. Headset,
comprising a first earpiece (2) and a second earpiece (3), wherein the first earpiece (2) and the second earpiece (3) are connected to one another by way of a connection element (4),
wherein the earpieces (2, 3) each have an inner side (5, 5') and an outer side (6, 6'), wherein the inner sides (5, 5') of the earpieces (2, 3) face towards one another and the outer sides (6, 6') of the earpieces (2, 3) face away from one another,
wherein the earpieces (2, 3) each extend from their bottom side (7, 7') to their top side (8, 8') in a vertical direction (30) and from their first longitudinal side (9, 9') to their second longitudinal side (10, 10') in a second transverse direction (32),
wherein the first earpiece (2) and/or the second earpiece (3) have/has, on their/its outer side (6, 6'), a positioning pocket (15, 15') with at least one button (22, 22'), wherein the positioning pocket (15, 15') extends over a width (a) in the second transverse direction (32) of the earpieces (2, 3), wherein the width (a) of the positioning pocket (15, 15') corresponds to at least 50% of the width (b) of the earpieces (2, 3) measured in the second transverse direction,
wherein the positioning pocket (15, 15') extends from a lower end (16, 16') to an upper end (17, 17') in the vertical direction (30) of the earpieces (2, 3), the upper end (17, 17') of the positioning tab (15, 15') is formed by a stop edge (20, 20') for abutment of the fingertips of an operator,
**characterized in that**, on the first earpiece (2) and/or on the second earpiece (3), a side button (27, 27') is arranged outside the positioning pocket (15, 15'), wherein the side button (27, 27') and the positioning pocket (15, 15') are arranged relative to one another in such a way that, in a basic position of the hand of the operator, the at least one button (22, 22') in the positioning pocket (15, 15') and the side button (27, 27') are actuatable, without the hand needing to be repositioned, and **in that**
the side button (27, 27') is arranged between the bottom side (7, 7') of the earpieces (2, 3) and the second longitudinal side (10, 10') of the earpieces (2, 3).

2. Headset according to Claim 1,
**characterized in that** the stop edge (20, 20') has a depth (f) measured in a first transverse direction from the inner side (5, 5') to the outer side (6, 6') of the earpieces (2, 3), wherein the depth (f) of the stop edge (20, 20') corresponds to at least 5% of the depth (c) of the earpieces (2, 3) measured in the first transverse direction (31).

3. Headset according to Claim 1 or 2,
**characterized in that** the positioning pocket (15, 15'), at its lower end (16, 16'), transitions without a step into a base surface (11, 11') forming the outer side (6, 6') of the earpieces (2, 3).

4. Headset according to one of Claims 1 to 3,
**characterized in that** the positioning pocket (15, 15') is delimited in the second transverse direction (32) of the earpieces (2, 3) by a first side wall (18, 18') and a second side wall (19, 19'), wherein the two side walls (18, 18', 19, 19') form a guide (21, 21') in the second transverse direction (32) of the earpieces (2, 3) for the lateral guidance of the fingers of the operator.

5. Headset according to one of Claims 1 to 4,
**characterized in that** only a connection button (23) is arranged in the positioning pocket (15, 15') of the first earpiece (2), said connection button extending over at least 50% of the width (a) of the positioning pocket (15, 15').

6. Headset according to Claim 5,
**characterized in that** the connection button (23) is designed for connection to a computer unit, in particular a smartphone.

7. Headset according to one of Claims 1 to 6,
**characterized in that** at least two buttons (24, 25, 26), in particular three buttons (24, 25, 26), are arranged in the positioning pocket (15, 15') of the second earpiece (3).

8. Headset according to Claim 7,
**characterized in that** the at least two buttons (24, 25, 26) are separated from one another by a separating web (28).

9. Headset according to Claim 7 or 8,
**characterized in that** one of the at least two buttons (24, 25, 26) is arranged in the positioning pocket (15, 15') so as to be recessed in relation to the other button (24, 25, 26) towards the inner side (5, 5') of the earpiece (3).

10. Headset according to Claim 9,
**characterized in that,** in the basic position of the hand of the operator, three buttons (24, 25, 26) arranged in the positioning pocket (15, 15') and the side button (27') are actuatable, without the hand needing to be repositioned.

11. Headset according to one of Claims 1 to 10,
**characterized in that**, when looking at the outer side (6, 6') in the first transverse direction (31), the earpieces (2, 3) have a first centroid (42) and the side button (27, 27') has a second centroid (43), wherein the distance (i), measured in the vertical direction (30), between the first centroid (42) and the second centroid (43) corresponds to at least 20%, in particular at least 30%, preferably approximately 35%, of the height (e) of the earpieces (2, 3).

12. Headset according to one of Claims 1 to 11,
**characterized in that** the side button (27) of the first earpiece (2) has three nublike elevations (41).

13. Headset according to one of Claims 1 to 12,
**characterized in that** the side button (27') of the second earpiece (3) is in the form of a circular function button, in particular a push-to-talk button or a mute button.

## Revendications

1. Casque,
comprenant un premier écouteur (2) et un deuxième écouteur (3), dans lequel le premier écouteur (2) et le deuxième écouteur (3) sont reliés l'un à l'autre par un élément de liaison (4),
dans lequel les écouteurs (2, 3) présentent respectivement une face intérieure (5, 5') et une face extérieure (6, 6'), les faces intérieures (5, 5') des écouteurs (2, 3) étant tournées l'une vers l'autre, et les faces extérieures (6, 6') des écouteurs (2, 3) étant détournées l'une de l'autre,
dans lequel les écouteurs (2, 3) s'étendent respectivement de leur face inférieure (7, 7') à leur face supérieure (8, 8') dans une direction verticale (30) et de leur première face longitudinale (9, 9') à leur deuxième face longitudinale (10, 10') dans une deuxième direction transversale (32),
dans lequel le premier écouteur (2) et/ou le deuxième écouteur (3) présente(nt) sur la face extérieure (6, 6') un logement de positionnement (15, 15') pourvu d'au moins une touche (22, 22'), le logement de positionnement (15, 15') s'étendant sur une largeur (a) dans la deuxième direction transversale (32) de l'écouteur (2, 3), dans lequel la largeur (a) du logement de positionnement (15, 15') correspond à au moins 50 % de la largeur (b) de l'écouteur (2, 3), mesurée dans la deuxième direction transversale,
dans lequel le logement de positionnement (15, 15') s'étend dans la direction verticale (30) de l'écouteur (2, 3) d'une extrémité inférieure (16, 16') à une extrémité supérieure (17, 17'), l'extrémité supérieure (17, 17') du logement de positionnement (15, 15') étant réalisée par un bord de butée (20, 20') pour poser le bout des doigts d'un utilisateur,
**caractérisé en ce que** qu'une touche latérale (27, 27') est disposée sur le premier écouteur (2) et/ou sur le deuxième écouteur (3) à l'extérieur du logement de positionnement (15, 15'), la touche latérale (27, 27') et le logement de positionnement (15, 15') étant disposés l'une par rapport à l'autre de telle sorte que dans une position de base de la main de l'utilisateur, ladite au moins une touche (22, 22') dans le logement de positionnement (15, 15') et la touche latérale (27, 27') peuvent être actionnées sans devoir repositionner la main, et **en ce que** la touche latérale (27, 27') est disposée entre la face inférieure (7, 7') de l'écouteur (2, 3) et la deuxième face longitudinale (10, 10') de l'écouteur (2, 3).

2. Casque selon la revendication 1,
**caractérisé en ce que** le bord de butée (20, 20') présente une profondeur (f) mesurée dans une première direction transversale de la face intérieure (5, 5') à la face extérieure (6, 6') de l'écouteur (2, 3), la profondeur (f) du bord de butée (20, 20') correspondant à au moins 5 % de la profondeur (c) de l'écouteur (2, 3), mesurée dans la première direction transversale (31).

3. Casque selon la revendication 1 ou 2,
**caractérisé en ce que** le logement de positionnement (15, 15') passe au niveau de son extrémité inférieure (16, 16') sans palier à une surface de base (11, 11') constituant la face extérieure (6, 6') de l'écouteur (2, 3).

4. Casque selon l'une quelconque des revendications 1 à 3,
**caractérisé en ce que** le logement de positionnement (15, 15') est limité dans la deuxième direction transversale (32) de l'écouteur (2, 3) par une première paroi latérale (18, 18') et une deuxième paroi latérale (19, 19'), les deux parois latérales (18, 18', 19, 19') formant une glissière (21, 21') dans la deuxième direction transversale (32) de l'écouteur (2, 3) pour le guidage latéral des doigts de l'utili sateur.

5. Casque selon l'une quelconque des revendications 1 à 4,
**caractérisé en ce que** dans le logement de positionnement (15, 15') du premier écouteur (2), seule est disposée une touche de liaison (23) qui s'étend sur au moins 50 % de la largeur (a) du logement de positionnement (15, 15').

6. Casque selon la revendication 5,
**caractérisé en ce que** la touche de liaison (23) est réalisée pour une liaison avec une unité de calcul, en particulier avec un smartphone.

7. Casque selon l'une quelconque des revendications 1 à 6,
**caractérisé en ce qu'au** moins deux touches (24, 25, 26), en particulier trois touches (24, 25, 26), sont disposées dans le logement de positionnement (15, 15') du deuxième écouteur (3).

8. Casque selon la revendication 7,
**caractérisé en ce que** les au moins deux touches (24, 25, 26) sont séparées les unes des autres par une barrette de séparation (28).

9. Casque selon la revendication 7 ou 8,
**caractérisé en ce que** l'une des au moins deux touches (24, 25, 26) est disposée dans le logement de positionnement (15, 15') en retrait par rapport à l'autre touche (24, 25, 26) en direction de la face intérieure (5, 5') de l'écouteur (3).

10. Casque selon la revendication 9,
**caractérisé en ce que** dans la position de base de la main de l'utilisateur, trois touches (24, 25, 26) disposées dans le logement de positionnement (15, 15') et la touche latérale (27') peuvent être actionnées sans devoir repositionner la main.

11. Casque selon l'une quelconque des revendications 1 à 10,
**caractérisé en ce que** dans la direction du regard le long de la première direction transversale (31) vers la face extérieure (6, 6'), l'écouteur (2, 3) présente un premier centroïde (42) et la touche latérale (27, 27') présente un deuxième centroïde (43), dans lequel la distance (i) mesurée dans la direction verticale (30) entre le premier centroïde (42) et le deuxième centroïde (43) correspond centroïde à au moins 20 %, en particulier à au moins 30 %, de préférence à environ 35 % de la hauteur (e) de l'écouteur (2, 3).

12. Casque selon l'une quelconque des revendications 1 à 11,
**caractérisé en ce que** la touche latérale (27) du premier écouteur (2) présente trois bossages (41) de type bouton.

13. Casque selon l'une quelconque des revendications 1 à 12,
**caractérisé en ce que** la touche latérale (27') du deuxième écouteur (3) est réalisée sous la forme d'une touche de fonction circulaire, en particulier d'une touche appuyer pour parler ou d'une touche de sourdine.
